# DEMANDE DE BREVET EUROPEEN

(11) **EP 4 306 112 A1**
(43) Date de publication de la demande: **17.01.2024**
(21) Numéro de dépôt: 22185116.5
(22) Date de dépôt: 15.07.2022
(51) Int. Cl.: A61K 31/498, A61P 17/16, A61K 31/201, A61K 31/355, A61K 9/00, A61K 9/06

(54) **NOUVEAU REGIME POSOLOGIQUE D'UNE COMPOSITION COMPRENANT DE LA BRIMONIDINE POUR SON UTILISATION DANS LA PREVENTION ET LE TRAITEMENT DES DOMMAGES CUTANES RESULTANT D'UN RAYONNEMENT**

(71) Demandeur: Tarian Pharma, 06130 Grasse (FR)
(72) Inventeur: Andres, Philippe, 06530 Peymeinade (FR); Czernielewski, Janusz, 06220 Vallauris (FR); Winckle, Gareth, 06160 Juan Les Pins (FR)
(74) Mandataire: Axe PI

(57) **Abrégé**

L'invention concerne une composition adaptée à une administration par voie topique comprenant de la brimonidine ou ses sels, pour son utilisation dans la prévention et le traitement des dommages cutanés résultant d'un rayonnement choisi parmi les ultraviolets (UV) incluant les UVA et UVB, les rayons dans le domaine du visible, le rayonnement infrarouge (IR), les rayonnements ionisants tels que les rayons X et les rayonnements alpha, beta, ou encore gamma ou les rayonnements composés de protons, en particulier d'un traitement par radiothérapie ou d'une exposition à un rayonnement UV. L'invention se caractérise en ce qu'au moins une dose efficace de ladite composition est administrée à un patient subissant un rayonnement plus d'1 heure et 24 heures avant une séance de rayonnement par application au niveau d'une zone recevant le rayonnement.

## Description

### Domaine technique

L'invention concerne le domaine des compositions pharmaceutiques. Elle concerne plus particulièrement une composition sous une forme adaptée à une administration par voie topique comprenant de la brimonidine ou ses sels, pour son utilisation dans la prévention et le traitement des dommages cutanés résultant d'un rayonnement. Deux types de rayonnement sont particulièrement visés, la radiothérapie responsable de radiodermites aigue ou chronique, et le rayonnement UV responsable de l'érythème actinique.

Parmi les autres types de rayonnements pouvant causer des dommages à la peau, on peut citer les autres longueurs d'onde du spectre solaire, notamment celles dans le domaine du visible ou de l'infrarouge (IR), les rayonnements ionisants tels que les rayons X et les rayonnements alpha, beta, ou encore gamma ou encore les rayonnements composés de protons.

Les données internationales indiquent par exemple que 50% des patients diagnostiqués avec un cancer sont amenés à recevoir une forme de radiothérapie. La radiodermite (ou dermatite radio-induite) est un effet secondaire important qui découle directement de l'exposition aux rayonnements pendant le traitement du cancer et concerne quasiment tous les patients cancéreux recevant une radiothérapie.

La radiodermite est qualifiée de réaction aigüe lorsqu'elle survient pendant le traitement et les quelques semaines qui suivent (par exemple 2-3 semaines) et d'apparition chronique ou tardive lorsqu'elle apparaît plusieurs mois (par exemple 3-6 mois) voire plusieurs années (jusqu'à par exemple 10 ans) après la fin du traitement. La radiodermite aigüe correspond à une réaction inflammatoire cutanée due à l'interaction des rayonnements ionisants avec la peau saine dans le champ d'irradiation. Le passage de rayons X au niveau de la peau induit dans les cellules des dégâts directs à l'ADN mais également la formation d'espèces réactives de l'oxygène (ROS) qui participent à la génération de dégâts cellulaires et pérennisent la réaction inflammatoire qui en découle. En effet, une vasodilatation secondaire des vaisseaux a lieu, permettant une migration et un passage accrus dans le tissu avoisinant des cellules et protéines sanguines inflammatoires responsables de la réaction inflammatoire locale cutanée. Le relargage parles cellules de cytokines pro-inflammatoires dont les interleukines 1 et 6 ainsi que TGF beta maintient l'inflammation. Les symptômes de la radiodermite aiguë ont été classés en quatre niveaux : grade 1 (érythème léger ou desquamation sèche), grade 2 (érythème modéré à vif ; desquamation suintante en plaque dans les plis et replis cutanés principalement ; oedème modéré) ; grade 3 (desquamation suintante confluente et étendue au-delà des plis ; saignement induit par des traumatismes ou abrasions mineurs), et grade 4 (ulcération, nécrose, hémorragie spontanée).

Parmi les patients qui reçoivent une radiothérapie, jusqu'à 95% peuvent présenter une forme de dermatite radio-induite. Ceci est notamment le cas dans le traitement des cancers du sein, du périnée et de la région de la tête et du cou.

Ces dernières années, les équipements modernes tels que la radiothérapie à modulation d'intensité ont réduit l'intensité de la dose sur la peau et de nouveaux équipements ont permis de réduire la gravité de la radiodermite aigüe chez de nombreux patients.

Néanmoins, la toxicité cutanée de grade 1 reste un problème pour environ 60% des patients et celle de grades 2-3 ou 4 pour environ 30% des patients.

Les radiodermites sont des lésions qui peuvent être prurigineuses, douloureuses ou suintantes. Elles peuvent produire un gonflement du sein et être génératrices d'anxiété. A ce titre, la radiodermite est le plus souvent responsable d'une altération de la qualité de vie. Néanmoins, en cas d'atteinte plus sévère, la radiodermite est susceptible d'entrainer une interruption thérapeutique temporaire, voir définitive, et donc de réduire la probabilité du contrôle tumoral.

La radiodermite chronique survient chez un tiers de tous les patients et peut apparaître jusqu'à 10 ans après le traitement de radiothérapie.

Les symptômes typiques sont les télangiectasies, des troubles de la pigmentation, l'atrophie cutanée (peau sèche et papyracée), la sclérose cutanée et les kératoses. La radiodermite chronique est donc un problème croissant notamment parce que plus de 80% de toutes les femmes traitées pour un cancer du sein survivent maintenant pendant au moins 10 ans ou plus.

Par ailleurs, l'érythème actinique, plus communément appelé coup de soleil, est une pathologie fréquente liée à l'exposition solaire et notamment aux rayons UV. Il se manifeste en général par un simple érythème rosé et cuisant qui va disparaître en quelques jours. Néanmoins, en fonction de l'intensité de l'exposition, il peut se présenter comme un érythème cyanique oedémateux douloureux avec desquamation et pigmentation durable, voire à la formation de phlyctènes avec altération de l'état général.

### Technique antérieure

Le traitement des radiodermites aigües ne fait pas l'objet d'un consensus. Différentes solutions ont été proposées, sans niveaux de preuve suffisants aujourd'hui pour être adoptées par tous et faire l'objet d'une recommandation.

Pour les radiodermites aigües de grade 1, des émollients, appliqués après la séance de rayons (par exemple DEXERYL^{®}, TOPICREME^{®}) hydratent la peau et apportent un bien-être transitoire au patient.

Il est important de noter que ces produits ne doivent pas être appliqués avant la séance, pour éviter un effet bolus (augmentation locale de la dose d'irradiation) et une aggravation des brûlures.

Certains produits plus spécifiques sont proposés sur les lésions de radiodermite tels que des crèmes à base d'acide hyaluronique (crème TETA^{®}) ou la BIAFINE^{®}. Il est important de noter ici que les études scientifiques ont démontré l'absence d'effet de ces produits comparativement à un placebo.

Les dermocorticoïdes locaux (par exemple DIPROSONE^{®}) sont à appliquer également après la séance. Le principe théorique d'utilisation de ces produits est de diminuer l'inflammation provoquée par la radiothérapie. Si les dermocorticoïdes n'apportent pas de réels bénéfices sur l'évolution des radiodermites, ils sont efficaces en cas de réaction allergique locale (par exemple en cas d'eczéma lié aux adhésifs utilisés pour les marquages).

Dans l'ensemble, ces traitements ne sont pas efficaces pour le traitement des radiodermites aiguës de grades 2, 3 ou 4.

Dans le cas de telles radiodermites aigües, la poursuite du traitement par radiothérapie peut être interrompue provisoirement si le radiothérapeute le juge nécessaire ou préférable, selon l'avancée du traitement et les priorités thérapeutiques.

Dans la mesure où le traitement est interrompu (par exemple 8 à 10 jours), des pansements vaselinés (par exemple GRASSOLIND^{®}) sont appliqués jusqu'à vingt-quatre heures avant la reprise de la radiothérapie, afin d'éviter qu'il reste des résidus gras. En cas de chimiothérapie concomitante et de défenses immunitaires affaiblies, des crèmes antibactériennes type FLAMMAZINE^{®} peuvent être ajoutées au protocole.

L'utilisation d'un vasoconstricteur topique, l'épinéphrine, a été décrite pour la prévention de la radiodermite chez des patientes atteintes d'un cancer du sein recevant une radiothérapie (James F. Cleary et al., Significant suppression of radiation dermatitis in breast cancer patients using a topically applied adrenergic vasoconstrictor. Radiation Oncology, 2017).

Toutefois, un tel produit, qui est une préparation extemporanée à base d'alcool qui s'évapore, requiert ainsi d'être appliqué juste avant le traitement par radiothérapie, jusqu'à 20 minutes avant. Son activité pharmacologique est limitée dans le temps et son efficacité est imparfaite avec environ 50% des patients seulement rapportant un bénéfice limité.

Aujourd'hui, il n'y a donc pas de solution particulièrement satisfaisante pour le traitement et/ou la prévention de la dermatite résultant d'un traitement par radiothérapie.

Dans ces traitements anticancéreux déjà difficiles à supporter pour les patients et dans lesquels les effets secondaires peuvent être limitants et venir affecter le déroulement optimal du protocole de traitement, il existe un réel besoin de disposer de nouveaux produits efficaces permettant de réduire significativement les effets secondaires cutanés de la radiothérapie.

Pour l'érythème actinique, la même approche d'une utilisation de cosmétiques émollients est proposée, sans efficacité sur l'évolution de la pathologie. Les anti-inflammatoires et l'aspirine à forte dose sont proposés dans les formes plus sévères mais ces traitements ne sont pas dénués d'effets indésirables.

Il existe donc un besoin de mettre au point de nouvelles formulations pour la prévention et/ou le traitement des dommages cutanés causés par les rayonnements et visant à réduire le flux sanguin dans la peau de manière prolongée et contrôlée dans le temps tout en limitant cet effet à la région concernée.

A cet effet, il est connu que l'application cutanée sur le visage d'un agoniste des récepteurs alpha2-adrénergiques hautement sélectif va réduire l'érythème par une vasoconstriction cutanée directe. La principale caractéristique de la vasoconstriction cutanée est la pâleur ; en diminuant le diamètre des artérioles et des petits vaisseaux du derme, cela entraine une réduction immédiate du flux sanguin produisant notamment une perte de la couleur de la peau.

Ainsi, le gel MIRVASO^{®} (0,5% p/p de brimonidine tartrate) est indiqué dans le traitement symptomatique de l'érythème facial associé à la rosacée chez l'adulte. La brimonidine est plus particulièrement connue comme étant un agoniste des récepteurs alpha2-adrénergique hautement sélectif. La brimonidine est 1 000 fois plus sélective pour les récepteurs alpha2-adrénergiques que pour les récepteurs alpha1-adrénergiques.

La brimonidine s'est avérée être utile dans le traitement de l'érythème causé par l'acné rosacée, et a été proposée pour d'autres troubles cutanés, voir par exemple la demande de brevet US10/853 585, la demande de brevet US10/626 037 et la demande de brevet US12/193098.

La brimonidine (tartrate) présente ainsi une stabilité chimique appropriée à une administration par voie topique et un profil de solubilité qui offre différentes options de formulation.

Cependant, l'action de cette molécule est limitée dans le temps (quelques heures) après une application topique. Il est en effet généralement plus difficile de créer une libération prolongée et des réservoirs cutanés avec des actifs ionisés y compris des sels car ils ont tendance à pénétrer moins facilement dans le stratum corneum (barrière composée majoritairement de lipides). De tels actifs ont également tendance à être rapidement éliminés des tissus viables en raison de leur solubilité aqueuse.

La brimonidine est en effet une molécule hydrophile et présente donc une pénétration difficile à travers le stratum corneum, lipidique.

Par contre, une fois le stratum corneum traversé, la brimonidine se trouve dans un milieu hydrophile (épiderme, notamment la couche granuleuse et la couche basale puis derme), pour être alors éliminée entraînant donc une perte d'efficacité en termes de vasoconstriction.

La formulation du gel MIRVASO^{®} à base de parahydroxybenzoate de méthyle, propylène glycol, carbomère, phénoxyéthanol, glycérol, titane dioxyde, sodium hydroxyde et d'eau purifiée n'est pas adaptée à la prévention de la radiodermite ou de l'érythème actinique; une telle formulation possède une pharmacocinétique inappropriée avec une activité limitée de 6 heures à 12 heures après application sur le visage et une durée encore réduite lorsque le produit est appliqué sur une autre partie du corps. Or dans ces pathologies, il est critique d'assurer une protection de 16 heures à 24 heures pour un résultat optimal. De plus, Mirvaso^{®} contient des particules de dioxyde de titane qui interfèrent avec les photons de la radiothérapie avec le risque de réduire son efficacité anti-tumorale.

A titre d'exemple illustratif, on connaît la publication Bouvier et *al.* (« Protective Effect of Dermal Brimonidine Applications Against UV Radiation-induced Skin Tumors, Epidermal Hyperplasia and Cell Proliferation in the Skin of Hairless Mice », 2015) qui divulgue l'application de compositions comprenant 0,2% et 2% de brimonidine tartrate dissoute dans du polyéthylèneglycol 400/éthanol/sérum physiologique - 30/20/50% w/w%) sur la partie haute du dos de souris (10 cm²) à t0, la souris étant soumise à une exposition UVB 1h après cette administration ou immédiatement avant cette administration. L'hyperplasie épidermique et la prolifération cellulaire ont ensuite été analysées.

D'après les résultats ainsi obtenus, et tel que conclu par les auteurs, la brimonidine a un plus grand potentiel lorsqu'elle est appliquée juste après le traitement UVB chez des souris glabres à une concentration de 2% (qu'à 0,2% et/ou par administration 1h avant un traitement UVB) au niveau de l'hyperplasie épidermique et de la prolifération cellulaire.

Il existe donc aujourd'hui un besoin certain de développer de nouvelles compositions permettant de limiter les dommages cutanés causés par les rayonnements, en particulier les effets secondaires cutanés du traitement du cancer par radiothérapie ou l'inflammation aigue résultant d'un coup de soleil.

Ce besoin est d'autant plus important que les cas de cancer continuent d'augmenter notamment en raison du vieillissement de la population.

### Problème technique

Considérant ce qui précède, un problème que se propose de résoudre la présente invention consiste à développer une formulation topique optimisée à base de brimonidine ou ses sels pour la prévention et le traitement des dommages cutanés résultant d'un rayonnement, en particulier d'un traitement par radiothérapie ou d'un érythème actinique.

Ce produit permettrait de répondre à un important besoin médical non satisfait en oncodermatologie, à la fois pour les cancérologues mais également pour les patients cancéreux. Ce produit viserait à améliorer l'efficacité d'un vasoconstricteur appliqué sur la peau pour prévenir et réduire de manière significative les principaux effets secondaires cutanés du traitement du cancer par radiothérapie. Cette amélioration comprendrait une activité rapide, puissante et prolongée, permettant une grande flexibilité dans les administrations (ce qui favoriserait significativement l'observance au traitement) mais également une plus grande efficacité.

Pour ce faire, il convient d'obtenir d'une part une traversée rapide pour induire une vasoconstriction quelques minutes après l'administration et avant la session de radiothérapie et le passage des rayons. Cette vasoconstriction doit être intense pour que la formation d'espèces réactives de l'oxygène (ROS) lors du passage des rayons dans la peau soit prévenue. Enfin la vasoconstriction doit être prolongée pendant 16 heures voire 24 heures, pour une prévention optimale de la réaction inflammatoire secondaire au stress oxydatif lié au rayonnement.

Afin d'assurer une protection optimale de la peau, le traitement doit être administré au moins une fois avant la première séance de radiothérapie.

### Avantages apportés

Une vasoconstriction locale et temporaire induite au niveau de la peau, avant irradiation par les rayonnements, en particulier les rayons de la radiothérapie, permet de réduire d'un côté, la quantité d'oxygène et de l'autre la migration et le passage des cellules et des protéines sanguines dans le tissu. Ceci conduit à une réduction des espèces réactives de l'oxygène et une réduction des processus inflammatoires, et donc la prévention totale ou partielle de la radiodermite.

Le Demandeur a développé une nouvelle composition topique qui délivre une durée de vasoconstriction améliorée en permettant une biodisponibilité du vasoconstricteur dans le derme et l'épiderme pendant une période supérieure à 12-14 heures, pour fournir une protection préventive et prolongée à la peau contre les dommages cutanés induits par les rayonnements et plus particulièrement contre les effets secondaires cutanés d'un traitement anticancer tout en évitant une interférence avec les rayons de la radiothérapie qui réduirait l'efficacité de ceux-ci sur la tumeur traitée ou le champ d'irradiation.

Des combinaisons complexes et difficiles à prévoir de solvants polaires de faible poids moléculaire (inférieur à 150 g/mol, préférentiellement inférieur à 100 g/mol) et de solvants polaires de plus gros poids moléculaire (supérieur à 150 g/mol, préférentiellement entre 350-650 g/mol) ont été avantageusement développées afin de créer un réservoir d'un vasoconstricteur hydrophile à la surface de la peau et dans les couches supérieures du stratum corneum. Or, la formation de réservoirs de composés hydrophiles est plus complexe que pour les agents lipophiles car les composés polaires ne se répartissent pas dans le stratum corneum aussi facilement que des composés plus lipophiles. En outre, les composés hydrophiles se répartissent plus librement dans les tissus viables et sont éliminés par la circulation du sang dans le système vasculaire local sous-jacent. Ainsi, l'identification d'une composition optimale est nécessaire pour moduler les différentes variables affectant la pharmacocinétique du produit notamment en termes de thermodynamique, solubilité superficielle résiduelle, solubilité dans le stratum corneum et pénétration dans les tissus viables (effets de traction / traînée de solvant).

La composition utilisée selon l'invention va permettre la création d'un réservoir d'actif vasoconstricteur polaire au niveau du stratum corneum, phénomène habituellement obtenu avec des molécules lipophiles telles que des corticostéroïdes, les molécules polaires étant généralement rapidement « lessivées ». Ceci permet une persistance prolongée dans la peau et un effet maximisé lors de l'application subséquente. Cette rémanence offre par ailleurs une plus grande flexibilité d'utilisation aux patients et aux radiothérapeutes notamment parce que l'application peut se déconnecter de l'horaire de la séance de radiothérapie.

La composition topique optimisée ainsi utilisée par le Demandeur améliore la durée et la puissance de vasoconstriction (d'une durée d'au moins 14 heures jusqu'à 24 heures) et ne perturbe pas la délivrance notamment des rayons sur la peau.

En outre, la concentration en vasoconstricteur est choisie pour obtenir une vasoconstriction présentant un effet rapide, constant et durable jusqu'à 16-24 heures tout en prévenant tout risque d'exposition systémique néfaste. Ces caractéristiques nouvelles permettent d'obtenir un état de vasoconstriction permanente seule à même de s'opposer efficacement aux phénomènes déclenchés par les séances répétées de radiothérapie et qui vont s'accumuler au fil des séances pour aboutir à la radiodermite.

L'optimisation de la composition est particulièrement adaptée au traitement par radiothérapie et permet de favoriser l'observance du patient et de maximiser l'efficacité du traitement anticancéreux. Il a ainsi été montré que l'efficacité vasoconstrictrice était augmentée et plus rapide lors de la deuxième administration. Cet effet surprenant permet de réduire le temps nécessaire pour obtenir la vasoconstriction puissante nécessaire au moment de la séance de radiothérapie. Cette découverte est à la base du régime posologique nouveau proposé avec une première administration du produit la veille de la première séance de radiothérapie et une seconde application le matin de la première séance de radiothérapie.

En plus des paramètres décrits ci-dessus, les éléments qui sont nécessaires pour créer des formes posologiques et des produits finis acceptables ont également été pris en compte lors du développement des compositions utilisées selon l'invention. Les compositions utilisées selon l'invention ont été avantageusement établies autour de systèmes de solvants optimaux qui facilitent l'administration cutanée et fournissent une stabilité physique, chimique et microbiologique adéquate, en plus d'une tolérance locale appropriée et d'une élégance cosmétique.

Aussi, la nouvelle formulation topique utilisée ainsi développée par le Demandeur est bien tolérée car non-irritante ou très faiblement irritante par rapport aux compositions de l'art antérieur, avec une pénétration cutanée et une solubilisation du vasoconstricteur améliorées.

Enfin, les compositions pharmaceutiques utilisées selon l'invention mises au point sont également économiques, faciles et rapides à préparer.

### Solution technique

La solution à ce problème posé a pour objet une composition adaptée à une administration par voie topique comprenant de la brimonidine ou ses sels, pour son utilisation dans la prévention et/ou le traitement des dommages cutanés résultant d'un rayonnement choisi parmi les ultraviolets (UV) incluant les UVA et UVB, les rayons dans le domaine du visible, le rayonnement infrarouge (IR), les rayonnements ionisants tels que les rayons X et les rayonnements alpha, beta, ou encore gamma ou les rayonnements composés de protons, caractérisée en ce qu'au moins une dose efficace de ladite composition est administrée à un patient subissant ledit rayonnement entre plus d'1 heure et 24 heures avant une séance de rayonnement par application au niveau d'une zone recevant ledit rayonnement.

### Brève description des dessins

L'invention et les avantages qui en découlent seront mieux compris à la lecture de la description et des modes de réalisation non limitatifs qui suivent, au regard des figures annexées dans lesquelles :
La Figure 1 représente les scores de blanchiment de la peau obtenus avec différentes compositions hydrogels 19-0155.0101/F1, 19-0155.0111/F1 et 19-0155.0112/F1.
La Figure 2 représente les scores de blanchiment de la peau obtenus avec des produits vasoconstricteurs de référence.
La Figure 3 représente les scores de blanchiment de la peau obtenus de manière comparative entre une composition hydrogel selon l'invention et une composition MIRVASO modifiée 1,5% p/p de brimonidine tartrate (19-0155-0098/F1) ainsi qu'avec une solution Norepinephrine et le produit MIRVASO^{®} (0,5% p/p de brimonidine tartrate).
La Figure 4 représente les scores de blanchiment de la peau obtenus sur une période de 72h avec différentes compositions hydrogels appliquées toutes les 24h.
La Figure 5 représente les scores de blanchiment de la peau obtenus sur une période de 72h avec une même composition hydrogel appliquée toutes les 12h.
La Figure 6 représente les scores de blanchiment de la peau obtenus sur une durée de 36h avec différentes posologies d'application de deux doses d'une même composition avant un traitement par radiothérapie.
La Figure 7 représente les scores moyens d'érythème obtenus avec différentes compositions actives (comprenant la brimonidine tartrate +/- un antioxydant) ou sans actif (ne comprenant que le véhicule +/- un antioxydant) appliquées la veille au soir et 2 heures avant l'irradiation UV.
La Figure 8 représente les scores moyens d'érythème obtenus avec une composition active (comprenant la brimonidine tartrate +/- BHA) ou sans actif (ne comprenant que le véhicule +/- BHA), lesdites compositions étant appliquées la veille au soir et 2 heures avant l'irradiation UV.
La Figure 9 représente le pourcentage de réduction de l'érythème obtenu avec une composition active (comprenant la brimonidine tartrate +/- BHA) ou avec le BHA pris seul par rapport au véhicule, lesdites compositions appliquées la veille au soir et 2 heures avant l'irradiation UV.
La Figure 10 représente les scores moyens d'érythème obtenus avec une composition active (comprenant la brimonidine tartrate +/- tocophérol) ou sans actif (ne comprenant que le véhicule +/- tocophérol), lesdites compositions appliquées la veille au soir et 2 heures avant l'irradiation UV.
La Figure 11 représente le pourcentage de réduction de l'érythème obtenu avec une composition active (comprenant la brimonidine tartrate +/- tocophérol) ou avec le tocophérol pris seul par rapport au véhicule, lesdites compositions appliquées la veille au soir et 2 heures avant l'irradiation UV.

### Description des modes de réalisation

L'invention concerne une composition adaptée à une administration par voie topique comprenant de la brimonidine ou ses sels, pour son utilisation dans la prévention et le traitement des dommages cutanés résultant d'un rayonnement choisi parmi les ultraviolets (UV) incluant les UVA et UVB, les rayons dans le domaine du visible, le rayonnement infrarouge (IR), les rayonnements ionisants tels que les rayons X et les rayonnements alpha, beta, ou encore gamma ou les rayonnements composés de protons, préférentiellement d'un traitement par radiothérapie ou d'une exposition à un rayonnement UV responsable par exemple de l'érythème actinique. Un traitement par radiothérapie désigne la délivrance de rayons, par exemple de rayons X ou de protons, à l'encontre d'une tumeur, de cellules tumorales ou d'un champ d'irradiation chez un patient donné.

L'invention se caractérise en ce qu'au moins une dose efficace de ladite composition utilisée selon l'invention est administrée à un patient subissant un rayonnement entre plus d'1 heure et 24 heures avant une séance de rayonnement par application au niveau d'une zone recevant ledit rayonnement, par exemple 2 heures, 3 heures, 4 heures, 5 heures, 10 heures, 15 heures, 20 heures et jusqu'à 24 heures, de préférence 2 heures, avant la première séance de rayonnement, préférentiellement un traitement par radiothérapie ou une exposition à un rayonnement UV.

De préférence, au moins une dose efficace de ladite composition utilisée selon l'invention est administrée à un patient subissant un traitement par radiothérapie entre plus d'1 heure et 24 heures avant une séance de traitement par radiothérapie par application au niveau de la zone recevant le traitement par radiothérapie pour réduire le flux sanguin dans la peau de manière prolongée et contrôlée dans le temps et dans la localisation restreinte au site d'application commun de ladite composition et dudit traitement par radiothérapie, par exemple 2 heures, 3 heures, 4 heures, 5 heures, 10 heures, 15 heures, 20 heures et jusqu'à 24 heures, de préférence 2 heures, avant la première séance de traitement par radiothérapie.

La composition utilisée selon l'invention est donc administrée au moins une fois avant la première séance de rayonnement, préférentiellement avant la première séance de traitement par radiothérapie ou d'exposition à un rayonnement UV.

Le vasoconstricteur utilisé dans la présente invention est la brimonidine ou ses sels. Par sels ou sels pharmaceutiquement acceptables, on entend les sels d'un composé d'intérêt qui sont sûrs et efficaces pour une utilisation topique chez des mammifères et qui possèdent une activité biologique souhaitée. Les sels pharmaceutiquement acceptables comprennent des sels de groupes acides ou basiques présents dans les composés spécifiés. Les sels d'addition acide pharmaceutiquement acceptables comprennent, mais ne sont pas limités à, des sels de chlorhydrate, bromhydrate, iodhydrate, nitrate, sulfate, bisulfate, phosphate, phosphate acide, isonicotinate, acétate, lactate, salicylate, citrate, tartrate, pantothénate, bitartrate, ascorbate, succinate, maléate, gentisinate, fumarate, gluconate, glucuronate, saccharate, formate, benzoate, glutamate, méthanesulfonate, éthanesulfonate, benzènesulfonate, ptoluènesulfonate et le pamoate (c'est- à-dire le 1,1'-méthylène-bis-(2-hydroxy-3-naphtoate)). Certains composés utilisés dans la présente invention peuvent former des sels pharmaceutiquement acceptables avec différents acides aminés. Des sels de base adaptés comprennent, mais ne sont pas limités à, des sels d'aluminium, calcium, lithium, magnésium, potassium, sodium, zinc, et diéthanolamine. Pour une revue sur les sels pharmaceutiquement acceptables, voir BERGE et al., 66 J. PHARM. SCI. 1 -19 (1977).

Dans le présent contexte, le terme « hydrate » désigne un composé d'intérêt, ou un sel pharmaceutiquement acceptable de celui-ci qui comprend en outre une quantité stoechiométrique ou non stoechiométrique d'eau liée à celui-ci par des forces intermoléculaires non covalentes.

De préférence, le sel de brimonidine utilisé dans les compositions utilisées selon l'invention est la brimonidine tartrate.

La composition utilisée selon l'invention comprend préférentiellement la brimonidine ou ses sels, de préférence la brimonidine tartrate, à une concentration comprise entre 0,15% et 1,50% en poids du poids total de la composition, préférentiellement comprise entre 0,75% et 1,50%, plus préférentiellement entre 1,00% et 1,50%, encore plus préférentiellement de 1,00% ou 1,50% p/p.

La concentration en brimonidine ou ses sels, de préférence en brimonidine tartrate, et la dose ainsi appliquée est avantageusement adaptée en fonction du site d'application.

En effet, la barrière constituée par la peau est plus épaisse notamment en termes de stratum corneum à traverser au niveau des pieds et des mains que du cuir chevelu, le reste du corps et en particulier la poitrine, présentant une épaisseur intermédiaire. Ainsi, pour une même dose, la concentration préférentiellement utilisée sur le corps, par exemple la poitrine, est avantageusement de 0,75-1,50% p/p, supérieure à celle utilisée au niveau du cuir chevelu et inférieure à celle utilisée au niveau des pieds et mains.

La composition utilisée selon l'invention peut être appliquée sur toutes les zones de la peau sur lesquelles les rayons, en particulier les rayons de la radiothérapie, par exemple les rayons X ou les protons, sont dirigés et par exemple le sein, le périnée ou encore la région de la tête et du cou.

Par dommages cutanés résultant d'un rayonnement et en particulier d'un traitement par radiothérapie on désigne plus particulièrement une radiodermite (ou dermatite radio-induite) ; par dommages cutanés résultant d'une exposition à un rayonnement UV, on désigne un érythème actinique.

La radiodermite est un effet secondaire important qui découle directement de l'exposition aux rayonnements pendant le traitement du cancer et concerne quasiment tous les patients cancéreux recevant une radiothérapie.

L'érythème actinique est lié à l'exposition solaire et notamment aux rayons UV. Il se manifeste par un simple érythème rosé et en fonction de l'intensité de l'exposition, il peut se présenter comme un érythème cyanique oedémateux douloureux avec desquamation et pigmentation durable, voire à la formation de phlyctènes avec altération de l'état général.

De préférence, afin de rendre la prévention encore plus efficace dès la première séance de rayonnement, en particulier de traitement par radiothérapie ou d'exposition à un rayonnement UV, la composition utilisée selon l'invention est administrée deux fois avant la première séance de rayonnement, de préférence deux fois avant la première séance de traitement par radiothérapie ou d'exposition à un rayonnement UV.

Il a ainsi été montré que l'efficacité vasoconstrictrice est augmentée et plus rapide lors de la deuxième administration. Cet effet particulièrement surprenant permet de réduire le temps nécessaire pour obtenir la vasoconstriction puissante nécessaire au moment de la séance de rayonnement, en particulier de radiothérapie.

A titre d'exemple de régime posologique particulièrement avantageux selon l'invention, on peut administrer une première fois la composition utilisée selon l'invention la veille de la première séance de rayonnement, en particulier de traitement par radiothérapie ou d'exposition à un rayonnement UV, par exemple le soir, par exemple à 20h, et une seconde fois le matin, par exemple à 7h, 8h, 9h ou 10h, et ce au moins plus d'une heure, par exemple 2 heures, 3 heures, 4 heures ou 5 heures, de préférence 2 heures, avant la première séance de rayonnement, en particulier de traitement par radiothérapie ou d'exposition à un rayonnement UV. De préférence, à titre d'exemple de régime posologique particulièrement avantageux selon l'invention, la composition utilisée selon l'invention est administrée au moins une fois, de préférence au moins deux fois, avant chaque séance de rayonnement, préférentiellement avant chaque séance de traitement par radiothérapie ou d'exposition à un rayonnement UV, plus préférentiellement pendant toute la durée du traitement par radiothérapie jusqu'à la dernière séance de traitement par radiothérapie.

Selon un mode de réalisation avantageux de l'invention, les compositions sont utilisées sous forme d'hydrogel, émulsion huile dans eau ou émulsion eau dans huile, préférentiellement hydrogel et émulsion huile dans eau.

Selon un mode de réalisation particulièrement avantageux de l'invention, la composition utilisée comprend une phase solvantée comprenant :
- du polyéthylène glycol (PEG) en combinaison avec du propylène glycol (PG) et/ou du diméthylsulfoxyde (DMSO) ;
- un copolymère Polyvinylpyrrolidone/Vinyl Acetate (KOLLIDON VA 64) comme agent filmogène hydrophile ;
- de la glycérine ;
et se présente sous forme d'hydrogel.

La composition hydrogel préférentiellement utilisée selon l'invention comprend avantageusement du polyéthylène glycol (PEG) en combinaison avec du propylène glycol et/ou du diméthylsulfoxyde (DMSO).

Les PEG de petits poids moléculaires jusqu'à PEG-600, tels que PEG-200, PEG-300, PEG-400, ou encore PEG-400 SR sont préférentiellement utilisés, plus préférentiellement PEG-400 et encore plus préférentiellement PEG-400 SR favorisant avantageusement la stabilité et la tolérance de la composition hydrogel selon l'invention en limitant le potentiel d'irritation, éliminant les impuretés polaires et réduisant ainsi l'interaction excipient-actif (brimonidine tartrate) et la dégradation ultérieure de l'actif.

Bien que le PEG-400 ou PEG-400 SR présente une pénétration relativement faible dans le stratum corneum en raison de son poids moléculaire et de sa polarité élevée (faible coefficient de partage), il est le PEG préférentiellement utilisé dans la composition hydrogel utilisée selon l'invention pour réduire la vitesse de précipitation de l'actif à la surface de la peau et dans les couches supérieures du stratum corneum, pour une solubilisation superficielle. Ceci favorise l'administration de manière prolongée de la brimonidine tartrate dans les couches viables de la peau et spécifiquement dans le système vasculaire du plexus dermique où se trouve le site cible de la brimonidine tartrate. Le PEG-400 ou PEG-400 SR présente une solubilité adéquate pour favoriser une meilleure rétention de la brimonidine tartrate en solution à la surface de la peau et dans les couches supérieures du stratum corneum.

De préférence, la composition hydrogel utilisée selon l'invention comprend le PEG à une concentration comprise entre 1% et 20% en poids du poids total de la composition, préférentiellement comprise entre 5% et 15%, plus préférentiellement de 10%.

Le propylène glycol (PG, 1,2-propanediol) est un liquide transparent, incolore et hygroscopique qui est largement utilisé comme solvant et conservateur dans une variété de formules pharmaceutiques parentérales et non parentérales.

La pénétration relativement rapide du PG à travers le stratum corneum et sa volatilité peuvent épuiser le véhicule résiduel de son solvant, augmenter l'activité thermodynamique de l'actif dans le véhicule et ainsi modifier la force motrice de diffusion. En outre, la pénétration et la perméation du PG peuvent également perturber la barrière lipidique du stratum corneum et par conséquent réduire la résistance diffusionnelle.

Le PG possède ainsi des propriétés physico-chimiques favorables en termes de pénétration et de perméation cutanées et est absorbé par voie cutanée. Par conséquent, les solutés qui sont facilement dissous par le PG (c'est-à-dire une affinité élevée pour le solvant/véhicule) peuvent avantageusement bénéficier d'une amélioration de leur pénétration cutanée via un mécanisme de résistance au solvant ou des effets de traction.

Même si des données pour divers composés sont décrites dans la littérature comme indiquant que la délivrance cutanée de composés pharmaceutiquement pertinents peut être améliorée par le PG, il n'est pas évident pour l'homme du métier de prédire ses caractéristiques en termes de stabilité, efficacité de perméation de l'actif (brimonidine tartrate) et tolérabilité lorsqu'il est utilisé dans un système complexe de solvant pour obtenir des compositions topiques selon l'invention qui soient efficaces, stables et pharmaceutiquement acceptables.

A *fortiori,* le PG est connu pour pénétrer la peau plus rapidement que la plupart des actifs et que donc la précipitation de l'actif à la surface de la peau va limiter sa durée d'action.

De plus, les concentrations d'utilisation *in vivo* du PG sont généralement limitées à environ 20% p/p ou moins, pour éviter des réactions d'irritation locales.

Le choix d'utiliser le PG comme solvant et amplificateur de pénétration dans les compositions hydrogels préférentiellement utilisées selon l'invention n'est pas facilement prévisible en raison de la solubilité et d'autres variables liées au véhicule. De préférence, la composition hydrogel utilisée selon l'invention comprend le PG à une concentration comprise entre 5% et 40% en poids du poids total de la composition, préférentiellement comprise entre 10% et 30%, plus préférentiellement entre 15% et 25%, encore plus préférentiellement de 20%.

Le DMSO est un solvant aprotique incolore, inodore, miscible à l'eau et hygroscopique. Il est connu pour ses capacités à dissoudre de nombreuses petites molécules polaires et non polaires en plus de plusieurs agents polymères ainsi que comme agent facilitant la pénétration de composés hydrophiles et lipophiles, y compris des agents antiviraux, des stéroïdes et des antibiotiques.

Plusieurs mécanismes potentiels de pénétration cutanée ont été décrits pour le DMSO, notamment :
- modification de la conformation intercellulaire de la kératine, de la forme hélicoïdale en feuillet β,
- déplacement de l'eau liée de la kératine,
- extraction des lipides cutanés,
- interaction avec les groupes lipidiques de la tête polaire entraînant une fluidité lipidique et une résistance diffusionnelle réduite,
- interaction avec les chaînes alkyles lipidiques dans le stratum corneum,
- répartition accrue du véhicule dans le stratum corneum via l'augmentation de la solubilité des perméants dans le domaine aqueux entre les bicouches lipidiques.
Il est ainsi connu que le DMSO pénètre et imprègne facilement la peau.

De manière surprenante, le DMSO peut également fournir un effet de traînée de solvant en termes d'administration d'actif par voie cutanée.

Les effets du DMSO dépendent de la concentration et généralement des systèmes de co-solvants contenant une concentration en DMSO supérieure à 60% p/p produisent une efficacité optimale.

De telles concentrations relativement élevées de DMSO peuvent toutefois provoquer notamment des érythèmes et des irritations.

Ces facteurs limitent l'utilisation du DMSO dans des compositions topiques et transdermiques à des concentrations élevées.

À une concentration plus faible, le DMSO peut être utilisé comme solubilisant d'actif au niveau du stratum corneum, par exemple à une concentration de 45% dans le produit analgésique topique PENNSAID^{®}.

Dans les compositions hydrogels utilisées selon l'invention, le DMSO est préférentiellement utilisé à une concentration limitée par rapport à celles de l'art antérieur précité, inférieure à 20% p/p, préférentiellement inférieure à 10% p/p, plus préférentiellement de l'ordre de 5% p/p.

De manière avantageuse, la solubilité relativement élevée de la brimonidine tartrate dans le DMSO couplée à une concentration en font un solvant préféré pour les compositions hydrogels utilisées selon l'invention.

Il est particulièrement avantageux dans le cadre des compositions hydrogels utilisées selon l'invention de contrôler le rapport PEG:PG et PEG:DMSO.

Des concentrations trop élevées pour l'association PEG en combinaison avec PG et/ou DMSO, supérieures à 50% p/p ont des incidences négatives en termes d'intensité de vasoconstriction de la brimonidine tartrate.

Selon un mode de réalisation préféré de l'invention, PEG et PG sont utilisés suivant un ratio de 1:1 à 1:5, préférentiellement 1:2.

Selon un mode de réalisation préféré de l'invention, PEG et DMSO sont utilisés suivant un ratio de 1:1 à 5:1, préférentiellement 2:1.

Selon un mode de réalisation particulièrement préféré des compositions hydrogels utilisées selon l'invention, le PEG est pris en combinaison avec le PG seulement, plus préférentiellement une combinaison PEG-400 SR et PG.

De préférence, la composition hydrogel utilisée selon l'invention comprend du polyéthylène glycol (PEG) à une concentration de 10% en poids du poids total de la composition en combinaison avec du propylène glycol (PG) à une concentration de 20% en poids du poids total de la composition.

La composition hydrogel préférentiellement utilisée selon l'invention comprend un copolymère Polyvinylpyrrolidone/Vinyl Acetate (KOLLIDON VA 64^{®}) comme agent filmogène hydrophile.

De préférence, la composition hydrogel utilisée selon l'invention comprend le copolymère Polyvinylpyrrolidone/Vinyl Acetate (KOLLIDON VA 64^{®}) à une concentration comprise entre 0,1% et 1,5% en poids du poids total de la composition, préférentiellement comprise entre 0,25% et 1,4%, plus préférentiellement entre 0,5% et 1,3%, plus préférentiellement encore entre 0,75 et 1,25, encore plus préférentiellement de 1%.

La composition hydrogel préférentiellement utilisée selon l'invention comprend de la glycérine.

La glycérine (glycérol) est un humectant bien connu qui peut augmenter la rétention d'eau dans le stratum corneum et améliorer l'hydratation.

La glycérine est également connue et utilisée pour renforcer le fonctionnement normal de la barrière cutanée, favoriser l'élasticité et la plasticité de la peau, améliorer la douceur de la peau et procurer des effets anti-irritants. La glycérine est en effet capable d'attirer l'eau dans le stratum corneum à partir de l'épiderme et de l'atmosphère.

En raison de sa polarité relativement élevée, la glycérine ne pénètre pas dans la peau au même degré et profondeur que le propylène glycol mais peut s'accumuler et former un réservoir dans les régions hydrophiles du stratum corneum et augmenter la teneur en eau.

L'interaction de la glycérine avec le stratum corneum, sa distribution cutanée et sa solubilité relativement élevée pour la brimonidine tartrate (2 fois supérieure à celle du propylène glycol) offrent des avantages en termes d'amélioration de l'administration cutanée et de prolongation de la pénétration cutanée.

De préférence, la composition hydrogel utilisée selon l'invention comprend la glycérine à une concentration comprise entre 1% et 20% en poids du poids total de la composition, préférentiellement comprise entre 2% et 15%, plus préférentiellement entre 3% et 10%, encore plus préférentiellement de 4%.

De manière particulièrement avantageuse, l'association de PG et de glycérine améliore la distribution de l'actif, préférentiellement la brimonidine tartrate dans le stratum corneum.

De plus, de manière avantageuse, le DMSO fournit une fonction similaire au PG mais avec une solubilité plus élevée pour la brimonidine tartrate. Ce différentiel de solubilité est un avantage pour l'administration cutanée.

De préférence, la composition hydrogel utilisée selon l'invention comprend en outre un agent gélifiant choisi parmi la gomme xanthane et de l'hydroxyethylcellulose (HEC), pris seuls ou en combinaison.

Préférentiellement, la composition hydrogel préférentiellement utilisée selon l'invention comprend la gomme xanthane et/ou la HEC à une concentration comprise entre 0,10% et 1,50% en poids du poids total de la composition, préférentiellement comprise entre 0,2% et 1%, plus préférentiellement entre 0,2% et 0,75%, encore plus préférentiellement respectivement de 0,2-0,5% pour la gomme xanthane et 0,3-0,5% pour la HEC préférentiellement pris en combinaison.

Un film mixte souple est ainsi avantageusement formé à la surface de la peau avec la gomme xanthane et/ou HEC et le copolymère Polyvinylpyrrolidone/Vinyl Acetate (KOLLIDON VA 64^{®}) en plus des autres solvants non volatils, PG et du PEG permettant de créer un réservoir de brimonidine, de préférence de brimonidine tartrate, et ainsi de ralentir la précipitation de la brimonidine et de prolonger sa durée d'action.

De préférence, la composition hydrogel utilisée selon l'invention comprend en outre un antioxydant naturel ou synthétique, ou un capteur de radicaux libres.

L'antioxydant est préférentiellement choisi parmi l'hydroxyanisole butylé (BHA), le DL-tocophérol, le butylhydroxytoluène (BHT), le propaldehyde, l'ascorbate palmitate, le glutathion, pris seul ou en mélange, préférentiellement le BHA et/ou le DL-tocophérol. L'antioxydant est préférentiellement utilisé dans les compositions hydrogels utilisées selon l'invention à une concentration comprise entre 0,1% et 4,0% en poids du poids total de la composition, plus préférentiellement entre 0,1% et 1,0%, encore plus préférentiellement de 0,1%.

De préférence, dans la mesure où la composition hydrogel préférentiellement utilisée selon l'invention comprend en outre un antioxydant, la composition hydrogel préférentiellement utilisée selon l'invention comprend en outre un polysorbate, préférentiellement le polysorbate 80 (TWEEN 80 SR^{®}) et/ou de l'huile de ricin hydrogénée polyoxyéthylénée 40 (KOLLIPHOR RH 40^{®}).

Préférentiellement, la composition hydrogel utilisée selon l'invention comprend moins de 5% de polysorbate, préférentiellement le polysorbate 80 (TWEEN 80 SR^{®}), en poids du poids total de la composition, plus préférentiellement 1,0-1,5%, encore plus préférentiellement 1,0%, et/ou moins de 3% d'huile de ricin hydrogénée polyoxyéthylénée 40 en poids du poids total de la composition, préférentiellement 1,0% d'huile de ricin hydrogénée polyoxyéthylénée 40.

Le capteur de radicaux libres est préférentiellement l'amifostine ou le tempol.

Le capteur de radicaux libres est préférentiellement utilisé dans les compositions hydrogels utilisées selon l'invention à une concentration comprise entre 0,1% et 3,0% en poids du poids total de la composition, par exemple de 2,5% p/p.

De préférence, la composition hydrogel utilisée selon l'invention comprend en outre de l'alcool oléique (KOLLICREAM OA^{®}) et de la vitamine E, pris seuls ou en combinaison.

L'incorporation d'une phase solvant hydrophile avec des solvants qui ont des propriétés hygroscopiques, humectantes et revitalisantes de la peau, y compris le PG et la glycérine, permet d'améliorer la solubilité de la brimonidine tartrate dans le stratum corneum et d'y augmenter la teneur en eau.

L'incorporation avantageusement d'antioxydants améliore la stabilité de l'actif. Selon un mode de réalisation particulièrement préféré, les compositions hydrogels utilisées selon l'invention ne comprennent pas de dioxyde de titane.

Les compositions hydrogels utilisées selon l'invention permettent ainsi d'améliorer et de prolonger l'administration cutanée de la brimonidine tartrate et de répondre aux besoins des patients avec une vasoconstriction locale adéquate et prolongée et une protection de l'épiderme et du derme supérieure par rapport aux espèces réactives de l'oxygène et des médiateurs inflammatoires.

De telles compositions utilisées selon l'invention sont faciles à appliquer et peuvent être appliquées sur les peaux potentiellement irritées.

Elles offrent un séchage rapide avec un résidu minime sur la peau.

Les compositions hydrogels préférentiellement utilisées selon l'invention ont un pH compris entre 4,0 et 6,0, préférentiellement entre 4,2 et 5,5, plus préférentiellement entre 4,3 et 5,0, encore plus préférentiellement de 4,5.

Les compositions hydrogels préférentiellement utilisées selon l'invention ont une viscosité comprise entre 50 cps et 3000 cps, préférentiellement entre 300 cps et 2800 cps, par exemple d'environ 500 cps, 1000 cps, 1500 cps, 1600 cps, 1700 cps, 1800 cps, 2000 cps, 2500 cps ou 2750 cps.

Selon un autre mode de réalisation de l'invention, la composition préférentiellement utilisée selon l'invention est sous forme d'émulsion huile dans eau comportant des cristaux liquides, et comprend la brimonidine ou ses sels, préférentiellement la brimonidine tartrate, dans une phase solvantée comprenant :
- du polyéthylène glycol en combinaison avec du propylène glycol ;
- un copolymère Polyvinylpyrrolidone/Vinyl Acetate (KOLLIDON VA 64^{®}) comme agent filmogène hydrophile ;
- de la glycérine ;
- un émulsifiant choisi parmi l'association PEG-75 stearate et glycéryl monostéarate et l'association polyoxyethylène-20 sorbitan monostearate (POLYSORBATE-60) et alcool cétostéarylique ;
- un acide oléique ou un alcool oléique, préférentiellement un alcool oléique ;
et une phase huileuse adaptée à l'obtention d'une émulsion huile dans eau comportant des cristaux liquides.

La phase huileuse d'une telle composition huile dans eau comportant des cristaux liquides préférentiellement utilisée selon l'invention comprend préférentiellement de l'alcool cétylique et de l'alcool stéarylique, pris seuls ou en combinaison, préférentiellement une combinaison d'alcool cétylique et d'alcool stéarylique, plus préférentiellement l'alcool cétylique, l'alcool stéarylique et l'alcool oléique, pris en combinaison, à une concentration comprise entre 1% et 15% en poids du poids total de la composition.

La phase huileuse d'une telle composition huile dans eau comportant des cristaux liquides préférentiellement utilisée selon l'invention comprend, à la place de l'alcool cétylique et de l'alcool stéarylique et préférentiellement en combinaison avec, un ester triglycéridique d'acides gras capryliques et capriques saturés de noix de coco / palmiste et de glycérol d'origine végétale, un polypropylène glycol (PPG)-11 stearyl ether, pris seuls ou en combinaison, préférentiellement pris en combinaison, plus préférentiellement à une concentration comprise entre 5% et 10% en poids du poids total de la composition.

### Exemples

La présente invention va maintenant être illustrée au moyen des exemples suivants :

### Exemple 1 : Evaluation de l'effet sur le blanchiment cutané de différentes compositions d'hydrogels à faible viscosité en utilisant un modèle de vasoconstriction in vivo

### Méthodologie du test de blanchiment de la peau

Les paramètres du modèle utilisé sont décrits ci-après.
- Produits appliqués de manière aléatoire, application à 8h00 du matin
- Application
   ∘haut de la poitrine,
   ∘ 60 microlitres de formulation à l'aide d'une pipette à déplacement positif,
   ∘ surface (10 cm2) définie à l'aide de joints toriques en plastique
   ∘ Massage 30 secondes et séchage post-application du produit de 10 min Évaluation
   ∘ 10 points dans le temps pour la notation:
      ∘ 1, 2, 3, 4, 8, 10, 12, 14, 16 et 24 heures après l'application
      ∘ Score de blanchiment de l'investigateur: échelle = 0 - 3 (3 = maximum)

Les compositions utilisées selon l'invention et détaillées dans le Tableau 1 ont été testées pour évaluer et confirmer leur effet sur le blanchiment cutané en réduisant le flux sanguin dans la peau de manière prolongée et contrôlée dans le temps et en présentant un effet vasoconstricteur jusqu'à 24 heures après application.

**Tableau 1 : Compositions des prototypes d'hydrogel à faible viscosité 19-0155.0100/F1, 19-0155.0101/F1, 19-0155.0111/F1 et 19-0155.0112/F1**

| **Composition** / **N° Formule-Lot** | **19-0155.0100** / **F1** | **19-0155.0101 / F1** | **19-0155.0111 / F1** | **19-0155.0112/ F1** |
|---|---|---|---|---|
| **Concept** | **Hydrogel à faible viscosité** | | | |
| **Taille du lot (g)** | **200** | **200** | **100** | **100** |
| **Justification de la formule** | Formule 059F1 + Gomme Xanthane | Formule 100/F1 sans NATROSOL et sans KOLLIDON | Formule 101/F1 + KOLLIDON | Formule 101F1 + KOLLIDON + 0,1% BHA + TWEEN 80 |
| **Ingrédient** | **% p/p** | | | |
| Brimonidine Tartrate | **1,5** | **1,5** | **1,5** | **1,5** |
| Eau purifiée | 61,63 | 63,3 | 62,3 | 57,2 |
| Propylene Glycol | 20 | 20 | 20 | 20 |
| Glycerine 4810 Vegetale | 4 | 4 | 4 | 4 |
| PEG-400 | 10 | 10 | 10 | 10 |
| Gomme Xanthane | 0,2 | 0,2 | 0,2 | 0,2 |
| KOLLIDON VA 64 | 1 | 0 | 1 | 1 |
| NATROSOL 250 HHX | 0,5 | | | |
| Phenoxyethanol | 1 | 1 | 1 | 1 |
| hydroxyanisole butylée | | | | 0,1 |
| TWEEN 80 | | | | 5 |
| NaOH Solution 10% | 0,17 | | 0,16 | |
| **Total** | 100,00 | 100,00 | 100,00 | 100,00 |
| **Aspect macroscopique T0** | Gel jaune, transparent, à faible viscosité | Gel jaune, transparent, à faible viscosité | Gel jaune, transparent, à faible viscosité | Gel jaune, transparent, à faible viscosité |
| T1 M 4°C | - | Idem que T0 | - | Idem que T0 |
| T1 M TA | - | Idem que T0 | - | Idem que T0 |
| T1M 40°C | - | Idem que T0 | - | Idem que T0 |
| **Aspect microscopique T0** | Pas de cristaux, pas d'autres points à noter | Pas de cristaux, pas d'autres points à noter | Pas de cristaux, pas d'autres points à noter | Pas de cristaux, pas d'autres points à noter |
| T1 M 4°C | - | Idem que T0 | - | - |
| T1 M TA | - | Idem que T0 | - | - |
| T1M 40°C | - | Idem que T0 | - | - |
| pH T0 | 4,6 | - | 4,73 | 4,4 |
| T1M 4°C | - | - | 4,60 | 3,94 |
| T1M TA | - | - | 4,76 | 4,02 |
| T1M 40°C | - | - | 4,53 | 3,84 |
| Commentaires | | | T=3M TA: GY4, 40°C: GY3^{∗} | |

| | | | | |
|---|---|---|---|---|
| TA - température ambiante ^{∗} - Normes de référence de couleur Ph.Eur. utilisées pour définir l'intensité de couleur des produits testés. | | | | |

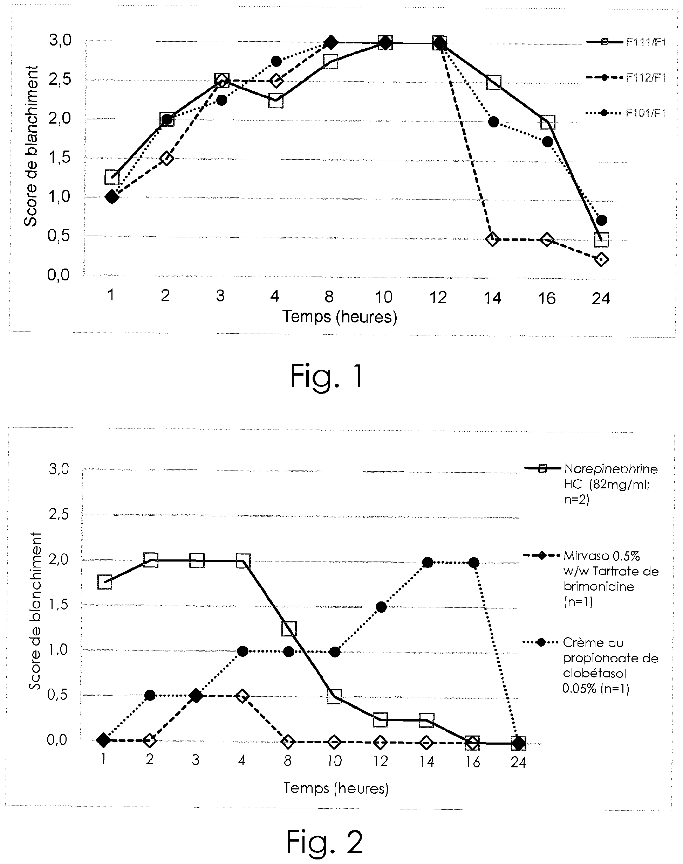

Tel qu'illustré par la Figure 1, chacune des compositions présente des profils de blanchiment de la peau similaires et atteint un score d'au moins 2 à 2.5 heures avec maintien de ce niveau de vasoconstriction pendant 13 à 16 heures. Ces compositions offrent donc des excellentes performances de blanchiment de la peau.

En revanche, la formule 19-0155.0112/F1 (F1 12/F1 dans la Figure 1) se comporte moins bien que les autres compositions avec un début plus lent et une durée de blanchiment plus courte. En fait, le blanchiment est revenu au niveau de base après 16 heures.

### Exemple 2 : Performance de produits de référence dans le modèle de blanchiment cutané

Le modèle de blanchiment cutané (vasoconstriction) tel que décrit à l'exemple 1 a été testé en utilisant des produits pharmaceutiques de référence connus pour induire un blanchiment de la peau/une vasoconstriction. Les études initiales ont été réalisées avec :
- Gel MIRVASO^{®} (0,5% p/p de brimonidine tartrate)
- Crème de propionate de clobétasol, 0,05% p/p
- Solution de chlorhydrate de norépinéphrine (82 mg / ml dans 70:30 éthanol:eau) telle qu'utilisée par Fahl (Effect of topical vasoconstrictor exposure upon tumoricidal radiotherapy. Int J Cancer; 135(4):981-988, 2014) et similaire à la formulation utilisée par Cleary et al. (Significant suppression of radiation dermatitis in breast cancer patients using a topically applied adrenergic vasoconstrictor. Radiation Oncology, 2017).

Le modèle de blanchiment de la peau permet de différencier la capacité de blanchiment de la peau provoqué par plusieurs actifs appliqués dans différentes formules en termes d'apparition, d'intensité et de durée du blanchiment de la peau. Le modèle présente une reproductibilité adéquate et des performances discriminatoires pour la phase de criblage de la formulation.

La crème de propionate de clobétasol a été sélectionnée comme produit/actif bien défini qui intervient dans le blanchiment de la peau. En fait, l'efficacité et la bioéquivalence *in vivo* des corticostéroïdes topiques (1997 FDA guidance, https://www.fda.gov/media/70931/download) est évaluée en utilisant cet effet de vasoconstriction.

Les données sur le blanchiment de la peau concernent le gel MIRVASO^{®}, la crème de propionate de clobétasol, 0,05% p/p et la solution d'épinéphrine HCl (82 mg / ml dans 70:30 Ethanol:eau) sont présentées graphiquement sur la Figure 2.

Le gel MIRVASO^{®} n'a pas généré de blanchiment significatif. Ceci était attendu car il a été conçu pour une application sur une peau du visage relativement fine et sensible dans le traitement de la rosacée. En règle générale, ces produits ne contiennent pas de fortes concentrations d'agents potentiels de pénétration cutanée en raison de la sensibilité cutanée des patients atteints de rosacée. De plus, la peau du visage est plus fine que la peau de la poitrine et possède ainsi une barrière inférieure à la pénétration et à la perméation cutanées. L'intensité et la durée d'action sont insuffisantes pour répondre aux exigences de la dermatite radio-induite souhaitées. La solution de norépinéphrine (noradrénaline) a produit un blanchiment cutané rapide et intermédiaire 1 heure après application ; cependant, l'effet a commencé à s'estomper après l'intervalle d'observation de 4 heures. Le blanchiment était à 0,5 ou moins après 10 heures et revenu au point de départ d'observation après 16 heures seulement. Alors que les effets initiaux étaient prometteurs, la durée et l'intensité de l'effet ne sont pas suffisantes.

Contrairement à la molécule polaire de norépinéphrine et à son véhicule éthanol aqueux, la crème de propionate de clobétasol a présenté un début d'action lent avec une augmentation progressive de 2 heures à un pic de score de blanchiment de 2,0 entre 14 et 16 heures. Il y a une réduction rapide du blanchiment à partir de 16 heures et retour au point de départ à 24 heures. Le début d'action plus lent du blanchiment au clobétasol peut être lié aux caractéristiques physico-chimiques de l'actif, par lequel son caractère lipophile se traduit par une formation de réservoir et une répartition plus limitée dans l'épiderme viable par rapport à la norépinéphrine plus hydrophile. Il est également important de noter que les mécanismes pharmacodymamiques de la vasoconstriction sont également différents pour le propionate de clobétasol et la norépinéphrine.

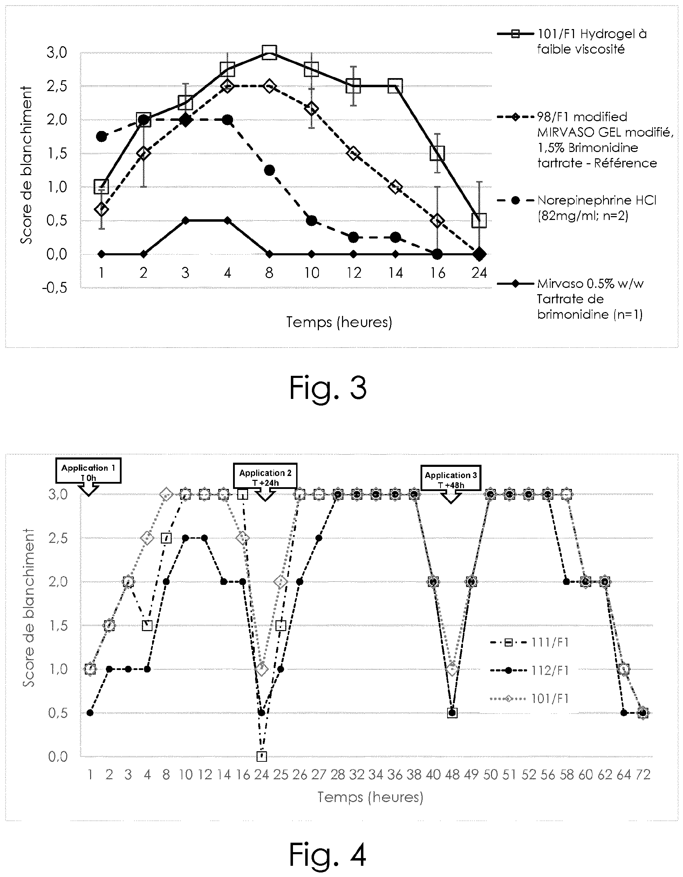

Aussi, une formule modifiée de type MIRVASO a été préparée avec une dose élevée de 1,5% p/p de brimonidine tartrate (19-0155-0098/F1). Cette évaluation a été menée pour évaluer l'impact d'une dose accrue sur le blanchiment de la peau dans un véhicule similaire à MIRVASO. Dans la même expérience, une composition hydrogel de faible viscosité selon l'invention (19-0155-101/F1, cf. Tableau 1) a également été testée à la même concentration. Les résultats de blanchiment de la peau correspondants pour les deux formules sont présentés sur la Figure 3. La solution de norépinéphrine et le gel MIRVASO^{®} disponible dans le commerce (brimonidine tartrate à 0,5%) sont également inclus à des fins de comparaison.

Les compositions ainsi testées sont détaillées dans le tableau ci-avant.

Le gel MIRVASO modifié (1,5% de Brimonidine tartrate 19-0155-0098/F1) a provoqué une augmentation substantielle de l'intensité et de la durée du blanchiment de la peau par rapport au gel MIRVASO^{®} commercialisé (0,5% de Brimonidine tartrate). Cependant, l'intensité du blanchiment de la peau n'a pas persisté pendant une durée prolongée comme souhaité pour résoudre le problème technique selon l'invention : l'atteinte d'un niveau de vasoconstriction à un niveau 2 nécessite 3 heures et ne se maintient que 10 heures, ce qui ne permet pas d'obtenir une protection adéquate. Cette limitation des performances est également couplée à l'inadaptation du véhicule MIRVASO dans la radiodermite. Comme indiqué précédemment, la présence de particules de dioxyde de titane dans le véhicule MIRVASO va interférer et perturber la dose de radiothérapie associée aux ondes électromagnétiques à haute énergie utilisées.

De manière surprenante, une formulation hydrogel à faible viscosité selon l'invention (19-0155-101/F1) a offert des performances améliorées par rapport au gel MIRVASO modifié (1,5% de Brimonidine tartrate 19-0155-0098/F1) en termes d'apparition d'action, effet de pointe et durée de l'action.

La formulation hydrogel à faible viscosité selon l'invention (19-0155-101/F1) a démontré une supériorité de performances substantielle tout au long de l'expérience, depuis la deuxième heure et jusqu'à la 24^{ème} (Figure 3).

La reproductibilité du modèle de blanchiment de la peau a été démontrée par le petit écart type associé aux trois répliques de formule 19-0155-101/F1 (Figure 3, résultats présentés comme la moyenne de trois répliques ± écart-type).

### Exemple 3: Evaluation de l'effet de différents régimes d'administration de compositions hydrogels utilisées selon l'invention

### 3.1 Résultats pour une dose administrée toutes les 24 heures pendant 72 heures

Ce régime d'application est considéré comme équivalent à l'application du produit une fois par jour pendant la période de traitement de radiothérapie.

Chaque formulation a été testée par application toutes les 24 heures pendant 72 heures et les résultats obtenus en termes de blanchiment de la peau sont illustrés par la Figure 4.

La formulation 19-0155.0112/F1 a produit un blanchiment de la peau légèrement inférieur aux formulations 19-0155.0101 /F1 et 19-0155.0111/F1.

Néanmoins, et de manière particulièrement surprenante et avantageuse, toutes les formulations ont démontré une réponse plus rapide et plus intense après les deuxième et troisième doses administrées. Après la première dose administrée, chaque formulation a atteint ses scores de blanchiment les plus élevés après 8 à 10 heures ; cependant, après les deuxième et troisième doses administrées, toutes les formulations ont atteint l'effet de blanchiment maximal sur l'échelle 0-3, 2 heures seulement après l'application. Cela représente un avantage certain en termes de flexibilité d'application de la composition dans la vie réelle à la fois pour le patient et pour le radiothérapeute.

Ces données indiquent qu'un réservoir s'est bien formé et est mobilisable lors des nouvelles applications, permettant d'atteindre plus rapidement l'activité vasoconstrictrice désirée.

### 3.2 Résultats pour une dose administrée toutes les 12 heures pendant 72 heures

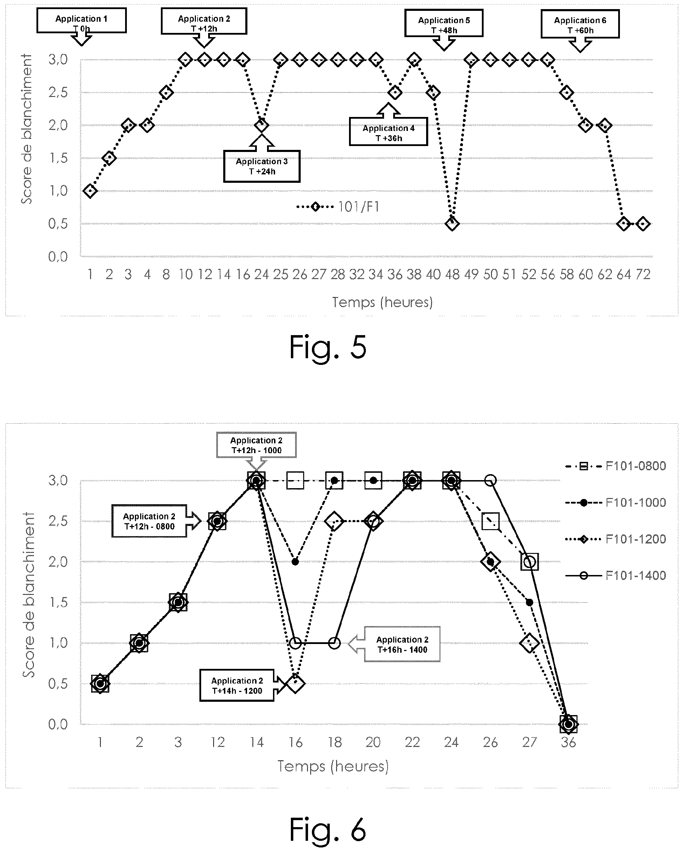

Afin de simuler une application biquotidienne d'une composition utilisée selon l'invention, une application toutes les 12 heures (à 8h00 et 20h00) pendant 72 heures a été réalisée. Une formulation utilisée selon l'invention a ainsi été plus particulièrement testée, 19-0155.0101/F1, et les résultats obtenus en termes de blanchiment de la peau sont illustrés par la Figure 5.

L'application avec une fréquence de 12 heures permet de générer un blanchiment de la peau plus complet sur la durée de l'expérience de 72 heures par rapport à la fréquence de dosage de 24 heures avec un maintien à un score de 3 encore plus constant.

### 3.3 Résultats pour des schémas d'utilisation assimilables à celles lors d'un traitement par radiothérapie

Le schéma posologique envisagé pour ce test a été conçu pour simuler un scénario de radiothérapie réel. Le cadre d'utilisation conçu pour cette expérience implique un prétraitement avec une formulation utilisée selon l'invention, 19-0155.0101/F1 (telle qu'utilisée précédemment) à 20h00 la nuit avant la radiothérapie avec une dose de suivi 2 heures avant la séance de radiothérapie prévue, à soit 08h00 (avec à 10h00 radiothérapie), 10h00 (avec à 12h00 radiothérapie), 12h00 (avec à 14h00 radiothérapie) ou 14h00 (avec à 16h00 radiothérapie). Les détails de dosage sont également décrits dans le Tableau 2.

**Tableau 2 : Détails de l'application pour chaque scénario envisagé**

| **Test ID** | **2000** | **0800** | **1000** | **1200** | **1400** | **1600** |
|---|---|---|---|---|---|---|
| **101/F1** - **0800** | 1ère dose | 2ème dose | Radiothérapie^{∗} | | | |
| **101/F1** - **1000** | 1ère dose | | 2ème dose | Radiothérapie^{∗} | | |
| **101/F1** - **1200** | 1ère dose | | | 2ème dose | Radiothérapie^{∗} | |
| **101/F1** - **1400** | 1ère dose | | | | 2ème dose | Radiothérapie^{∗} |

Les scores de blanchiment de la peau après application de la formulation utilisée selon l'invention, 19-0155.0101/F1, dans de telles conditions d'utilisation est illustré par la Figure 6.

Pour les scénarios d'application 101/F1 - 08h00 et 101/F1 - 10h00, c'est à dire avec une deuxième application à 8h ou à 10h (2 heures avant la séance de radiothérapie), le blanchiment maximal de la peau a été maintenu au niveau le plus élevé sur une durée prolongée.

Un score de blanchiment en baisse notable a été observé avec les 101/F1 - 12h00 et 101/F1 - 14h00 avant la 2ème dose; cependant, après administration de la deuxième dose, le blanchiment de la peau a renvoyé un score de blanchiment de 3 après 3-4 heures et a été maintenu pendant une période prolongée.

### 3.4 Conclusions quant à l'effet de la fréquence d'application d'une composition utilisée selon l'invention sur le blanchiment de la peau

Le schéma posologique impliquant une application toutes les 24 heures à 8 heures du matin, c'est-à-dire une application une fois par jour, a très bien fonctionné et donne des résultats très satisfaisants. La couverture de blanchiment de la peau est avantageusement améliorée par application 1 jour avant le début du traitement de radiothérapie pour assurer le blanchiment maximal de la peau (vasoconstriction) grâce à un effet réservoir engendré par les compositions utilisées selon l'invention. Aussi, le blanchiment de la peau a semblé saturer rapidement avec le schéma posologique de 12 heures (application deux fois par jour, 08h00 et 20h00) qui n'est donc pas utile.

Le schéma d'utilisation assimilable à un cas réel a révélé des résultats améliorés et optimaux avec les schémas 101/F1 -08h00 et 101/F1 - 10h00 (c'est-à-dire l'application un jour avant le début de la radiothérapie avec une deuxième application 2 à 4 heures avant la première séance de radiothérapie) semblant être encore plus efficace en termes de couverture du blanchiment de la peau.

Un régime posologique optimal de la composition utilisée selon l'invention a donc été obtenu en appliquant ladite composition la veille au soir puis 2 heures avant le traitement par radiothérapie et ce sur la durée du traitement.

### Exemple 4 : Evaluation de l'efficacité sur un modèle d'érythème induit par les UV

Des compositions actives et des véhicules provenant des trois principaux types de formulations (Hydrogel et 2 émulsions Gelot-64 et Polawax-NF, Tableau 3) ont été évalués à l'aide du modèle d'érythème induit par les UV.

Les compositions ont été appliquées en utilisant le protocole tel que spécifié ci-après. Cela implique, conformément aux travaux décrits ci-dessus, une application la veille au soir de l'irradiation UV et une nouvelle application 2 heures avant l'irradiation UV. Des doses érythémales minimales individuelles (MED = dose minimale d'UV nécessaire pour générer un érythème homogène sur l'ensemble de la zone irradiée) pour chaque sujet (3 sujets) ont été déterminées 24 heures avant l'expérience. Neuf mini-zones ont également été délimitées sur le dos de chaque sujet où les compositions ont été appliquées à une dose de 5 mg / cm2. Trois doses UV ont été administrées sur chacune des zones test: 1 x MED (1MED), 2 x MED (2MED) et 3 x MED (3MED).

Une lecture de la réaction érythémale a été effectuée 24 heures après l'irradiation en utilisant un score d'érythème par l'investigateur (0 - érythème absent, 3- érythème maximal) et en utilisant un colorimètre Chroma Meter.

**Tableau 3 :**

| **Forme de dosage** | **Description** | **Caractéristiques principales** | **N° Formule** | **N° Référence** |
|---|---|---|---|---|
| Gel | Hydrogel à faible viscosité | Brimonidine tartrate 1.5% + BHA 0.1% + 1.5% TWEEN 80 SR | 19-0155.0135/F1 (cf. Tableau 4) | A |
| | | Brimonidine tartrate 1.5% + 1.5% TWEEN 80 SR | 19-0155.0134/F1 (cf. Tableau 4) | B |
| | | Véhicule | 19-0155.0134P/F1 (véhicule de la formule 19-0155.0134/F1; sans actif brimonidine tartrate) | C |
| | | BHA 0.1% + 1.5% TWEEN 80 SR | 19-0155.0135P/F1 (cf. Tableau 4) | D |
| Émulsion | GELOT 64 Émulsion des cristaux liquides | Brimonidine tartrate 1.5% + BHA 1.0% | 19-0155.0102/F5 (cf. Tableau 5) | E |
| | | BHA 1.0% | 19-0155.0102P/F2 (cf. Tableau 5) | F |
| | POLAWAX NF - Émulsion des cristaux liquides | Brimonidine tartrate 1.5% + Tocopherol 0.1% | 19-0155.0132/F2 (cf. Tableau 6) | G |
| | | Tocopherol 0.1% | 19-0155.0132P/F1 (cf. Tableau 6) | H |
| | | Brimonidine tartrate 1.5% + Tocopherol 1.0% | 19-0155.0133/F1 (cf. Tableau 6) | I |

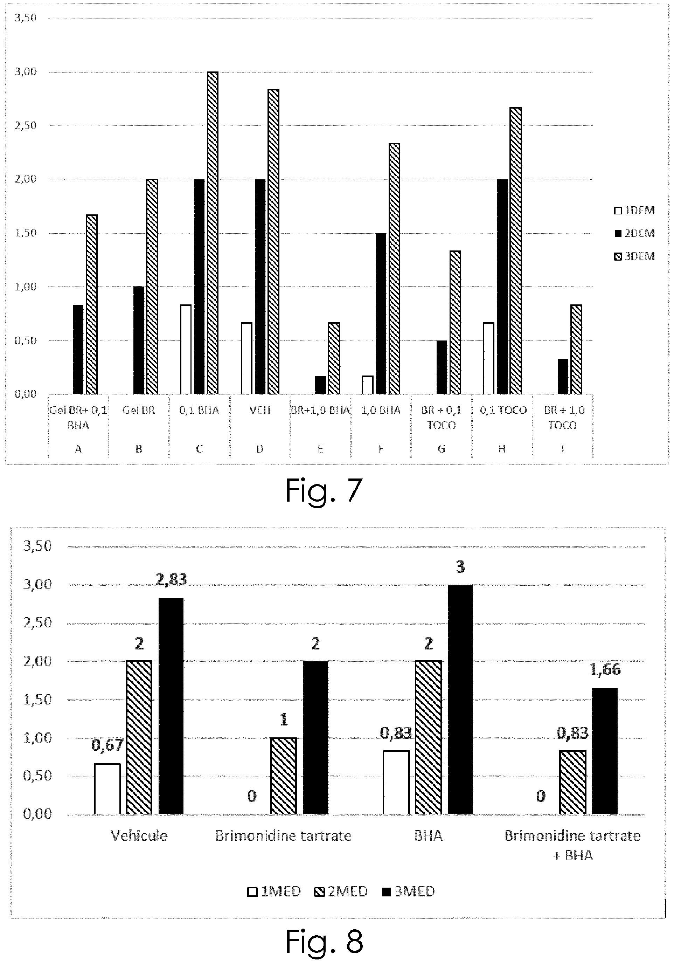

Les scores moyens d'érythème de l'investigateur pour chaque formulation sont résumés dans la Figure 7.

Le BHA ou le tocophérol (alpha-tocophérol) pris seul ne produisent aucun effet sur la réduction d'érythème.

Or, tel qu'illustré par la Figure 7, les formules actives contenant 1,5% de brimonidine tartrate ont démontré des réductions substantielles des scores d'érythème par rapport au véhicule ou par rapport au antioxydants utilisés seuls. Aussi, des bénéfices supplémentaires synergiques en termes d'effets anti-érythémateux ont été observés lorsque 1,5% p/p de brimonidine tartrate est associée à des antioxydants. Le BHA et l'aalpha-tocophérol ont été utilisés comme antioxydants modèles à des concentrations de 0,1% et 1% p/p. Les formulations contenant 1% de BHA ou 1% d'a-tocophérol ont démontré les effets anti-érythémateux les plus puissants et ont inhibé efficacement 2 MED. La formule 19-0155.0102/F5 (émulsion de cristaux liquides H/E GELOT 64) a généré l'effet anti-érythème le plus important.

Aussi, de manière surprenante, l'association de brimonidine tartrate avec un antioxydant, BHA ou tocophérol, produit un effet supérieur sur la réduction de l'érythème que la brimonidine tartrate prise seule indiquant un effet synergique entre les deux produits.

Les compositions hydrogels utilisées selon l'invention ont démontré des propriétés anti-érythémateuses efficaces dans le modèle d'érythème induit par les UV. Lorsque 1,5% p/p de brimonidine tartrate a été combinée avec des antioxydants, du BHA ou de l'alpha-tocophérol, des bénéfices supplémentaires synergiques ont été observés en termes d'anti-érythème. Les effets anti-érythémateux les plus puissants ont été observés lorsque la brimonidine tartrate est associée à 1,0% de chaque antioxydant.

### Exemple 5: Mise en évidence d'un effet synergique entre la brimonidine tartrate et le BHA sur la réduction de l'érythème sur un modèle d'érythème induit par les UV

Sur la base du modèle et du protocole décrits dans l'exemple 4, les scores moyens obtenus avec les compositions détaillées dans le Tableau 4, à savoir respectivement une composition active (comprenant la brimonidine tartrate +/- BHA, 19-0155.0135/F1 et 19-0155.0134/F1) ou sans actif (ne comprenant que le véhicule +/- BHA, 19-0155.0135P/F1 et 19-0155.0134P/F1) sont résumés dans la Figure 8.

**Tableau 4 : Compositions détaillées d'hydrogels à faible viscosité**

| **Composition / N° Formule-Lot** | **19-0155.0135/ F1** | **19-0155.0134 / F1** | **19-0155.0135P** / **F1** |
|---|---|---|---|
| **Concept** | **Hydrogel à faible viscosité** | | |
| **Taille du lot (g)** | 100 | 100 | 100 |

| **Justification de la formule** | Formule 121/F1 + 1,5% TWEEN 80 SR | Formule 111 /F1 + 1,5% TWEEN 80 SR | Formule 121/F1 véhicule + 1,5% TWEEN 80 SR |
|---|---|---|---|
| **Ingrédient** | % p/p | | |
| Eau purifiée | 60,49 | 60,54 | 62,01 |
| Glycérine 4810 Végétale | 4 | 4 | 4 |
| Propylène Glycol | 20 | 20 | 20 |
| Brimonidine Tartrate | 1,5 | 1,5 | |
| Gomme Xanthane | 0,2 | 0,2 | 0,2 |
| PEG-400 SR | 10 | 10 | 10 |
| BHA | 0,1 | | 0,1 |
| TWEEN 80 SR | 1,5 | 1,5 | 1,5 |
| DL Tocopherol | | | |
| KOLLIPHOR RH40 | | | |
| KOLLIDON VA 64 | 1 | 1 | 1 |
| KOLLIPHOR EL | | | |
| Phénoxyéthanol | 1 | 1 | 1 |
| Acide Citrique Solution 10% | | | 0,19 |
| NaOH 10% sol | 0,21 | 0,26 | |
| **Total** | 100.00 | 100.00 | 100.00 |
| **Données de stabilité** | | | |
| Aspect macroscopique T0 | Gel clair, jaune, de faible viscosité | Gel clair, jaune, de faible viscosité | Gel clair, incolore et de faible viscosité |
| Aspect microscopique T0 | Pas de cristaux, pas d'autres points à noter | Pas de cristaux, pas d'autres points à noter | Pas de cristaux, pas d'autres points à noter |
| pH T0 | 4,73 | 4,8 | 4,68 |

**Tableau 5 : Compositions détaillées des émulsions Gelot 64**

| **Composition** / **N° Formule-Lot** | **19.0155-0102/F4** | **19.0155-0102/F5** | **19.0155-0102P/F2** |
|---|---|---|---|
| **Concept** | **GELOT** | **GELOT** | **GELOT** |
| **Taille du lot (g)** | **200** | **200** | **200** |
| **Justification de la formule** | Formule 0090 + 1% BHA | Formule 0090 avec 1% BHA pour mise en stabilité dans flacons validés | Formule 0090P véhicule + 1% BHA |
| **Ingrédient** | **% p/p** | | |
| Brimonidine Tartrate | **1,5** | **1.5** | 0 |
| Eau purifiée | 39.82 | 39.8 | 41,5 |
| Propylène Glycol | 20 | 20 | 20 |
| Glycérine 4810 Végétale | 5 | 5 | 5 |
| PEG-400 SR | 10 | 10 | 10 |
| KOLLIDON VA 64 | 1 | 1 | 1 |
| GOMME XANTHANE | 0,2 | 0.2 | 0,2 |
| METHYL PARABEN | 0,2 | 0.2 | 0,2 |
| PHENOXYETHANOL | 1 | 1 | 1 |
| GELOT 64 | 3 | 3 | 3 |
| KOLLIWAX SA | 3 | 3 | 3 |
| MIGLYOL 812N | 2,5 | 2.5 | 2,5 |
| ARLAMOL PS11E-LQ-(RB) | 5 | 5 | 5 |
| KOLLICREAM OA | 2,5 | 2.5 | 2,5 |
| KOLLIWAX CA | 4 | 4 | 4 |
| BHA | **1** | **1** | 1 |
| PROPYL PARABENE | 0,1 | 0.1 | 0,1 |
| DL TOCOPHEROL | | | |
| NaOH 10% | 0,18 | 0.2 | |
| **Total** | 100.00 | 100.00 | 100.00 |
| **Données de stabilité** | | | |
| Aspect macroscopique T0 | Crème épaisse légèrement jaune qui ne coule pas | Crème jaunâtre qui ne coule pas | Crème blanche épaisse qui ne coule pas |
| T1M 4°C/TA/40°C | N/A | N/A | T1M Idem T0/ Idem T0 / Légère augmentation de la viscosité |
| T3M 4°C/TA/40°C | N/A | Idem T0 / légèrement plus teintée qu'à 4°C / légèrement plus teintée qu'à TA | N/A |
| Aspect microscopique T0 | Grand nombre de petits CL < 10µm. | Fond très fin et homogène, nombreux CL | Grand nombre de petits CL 5 - 10µm. |
| T1M 4°C/TA/40°C | N/A | N/A | T1M Idem T0/ Idem T0 quelques bulles / Idem T0 |
| T3M 4°C/TA/40°C | N/A | Idem T0 / idem T0, CL <10µm / Idem T0, bullée | N/A |
| pH T0 | 4,81 | 4,96 | 5,01 |
| T1M 4°C/TA/40°C | N/A | N/A | 4,77 / 4,70 / 4,74 |
| T3M 4°C/TA/40°C | N/A | 4,70 / 4,70 / 4,70 | N/A |
| Viscosité (RV mobile 34-6rpm- 5min) T0 | | mob RV29 vit 30: 6633cP / 19,9% (le 03/07/20) | |
| T3M 4°C / TA / 40°C | | TA: 10930 cP, 32,8% | |

| | | | |
|---|---|---|---|
| CL - Cristaux liquides | | | |

**Tableau 6 : Compositions détaillées des émulsions Polawax NF**

| **Composition** / **N° Formule-Lot** | **19.0155-0132/F2** | **19.0155-0132P** /F1 | **19.0155-0133/F1** |
|---|---|---|---|
| **Concept** | **POLAWAX** | **POLAWAX** | **POLAWAX** |
| **Taille du lot (g)** | **200** | **200** | **200** |
| **Justification de la formule** | 19-0155.0091/F1 + 0,1% Tocopherol | 19-0155.0091P/F1 + 0,1% Tocopherol (véhicule) | 19-0155.0091 /F 1 + 1% Tocopherol |
| **Ingrédient** | **% p/p** | | |
| Glycérine 4810 Végétale | 4 | 4 | 4 |
| Propylène Glycol | 20 | 20 | 20 |
| PEG-400 SR | 10 | 10 | 10 |
| Eau purifiée | 45,5 | 47,03 | 44,6 |
| Brimonidine Tartrate | **1,5** | | **1,5** |
| KOLLIDON VA 64 | 1 | 1 | 1 |
| GOMME XANTHANE | 0,2 | 0,2 | 0,2 |
| POLAWAX NF | 10 | 10 | 10 |
| ARLAMOL PS11 E-LQ-(RB) | 2,35 | 2,35 | 2,35 |
| DIMETHICONE (XIAMETER PMX-200) | 1 | 1 | 1 |
| KOLLICREAM OA | 2,5 | 2,5 | 2,5 |
| MARCOL 82 | 0,65 | 0,65 | 0,65 |
| BHA | | | |
| DL TOCOPHEROL | **0,1** | **0,1** | **1** |
| IP PARABENES | 1 | 1 | 1 |
| NaOH 10% | 0,2 | | 0,2 |
| Acid citrique Solution 10% | | 0,17 | |
| Total | 100.00 | 100.00 | 100.00 |
| **Données de Stabilité** | | | |
| Aspect macroscopique T0 | Légèrement jaune. Crème lisse et brillante qui ne coule pas | Crème blanche à blanc cassé, lisse et brillante, qui ne coule pas | Légèrement jaune. Crème lisse et brillante qui ne coule pas |
| T1M 4°C/TA/40°C | T1M Idem T0/ Epaississement en surface, effet nacré / couleur jaune en surface, non-homogène | T1M Idem T0 / Aspect blanc mat en surface / Idem T0 | T1M Idem T0 / aspect nacré, fines marbrures / surface plus épaisse et plus colorée |
| T2M 4°C/TA/40°C | N/A | T2M Idem T0/marbrures aspect nacré/crème épaisse, perte d'homoaénéité | T2M idem T0/ idem T1M / Idem T1M |
| T3M 4°C/TA/40°C | N/A | T3M Idem T0/Idem T2M/Idem T2M | T3M idem T1 M/ Anneau jaune en surface |
| Aspect microscopique T0 | Beaucoup de petits CL | Fond très fin, homogène. Globules <10µm. Beaucoup de petits CL. | Fond très fin, beaucoup de petits CL <10µm |
| T1M 4°C/TA/40°C | T1M idem T0/ idem T0/ idem T0 | T1M Idem T0/petits CL moins nombreux, qq gros globules ~20µm/Idem T1M à TA | T1M Idem T1M à TA/CL moins nets et certains déformés, fond très fin/formule bullée, gros CL déformés |
| T2M 4°C/TA/40°C | N/A | T2M Fond très fin/peu de bulles, idem T1 M/Bulles, fond hétérogène, CL moins nets | T2M idem T1 M/ idem T1 M/ fond très fin bullé, petits CL moins nets |
| T3M 4°C/TA/40°C | N/A | T3M Idem T2M/Idem T1 M/Hétérogène | T3M idem T1 M/ idem T1 M/ Perte d'homogénéité |
| pH T0 | 4,75 | 4,8 | 4,84 |
| T1M 4°C/TA/40°C | 4,88 / 4,78 / 4,98 | 4,78 / 4,68 / 4,92 | 4,93 / 4,83 / 5,03 |
| T2M 4°C/TA/40°C | N/A | 4,93 / 4,91 / 5,19 | 4,81 / 4,85 / 4,98 |
| T3M 4°C/TA/40°C | N/A | 4,91 /4,82 / 4,99 | 4,83 / 4,85 / 4,90 |

| | | | |
|---|---|---|---|
| CL - Cristaux Liquides | | | |

Tel qu'illustré par la Figure 8, en comparant les scores moyens d'érythème obtenus pour les quatre compositions testées, un effet (réduction de l'érythème) a pu être obtenu avec la composition comprenant de la brimonidine tartrate et un effet synergique peut même être observé avec la composition comprenant à la fois de la brimonidine tartrate et du BHA par rapport aux compositions comprenant la brimonidine tartrate ou le BHA pris seul.

En effet, après une irradiation 3 x MED, l'érythème a été diminué de 29% avec la composition comprenant la brimonidine tartrate sans antioxydant, et légèrement augmenté par la composition comprenant le BHA seul (sans brimonidine tartrate) (+ 6%), alors que la combinaison de brimonidine tartrate et de BHA a conduit de manière surprenante à une diminution de 41 % de l'érythème.

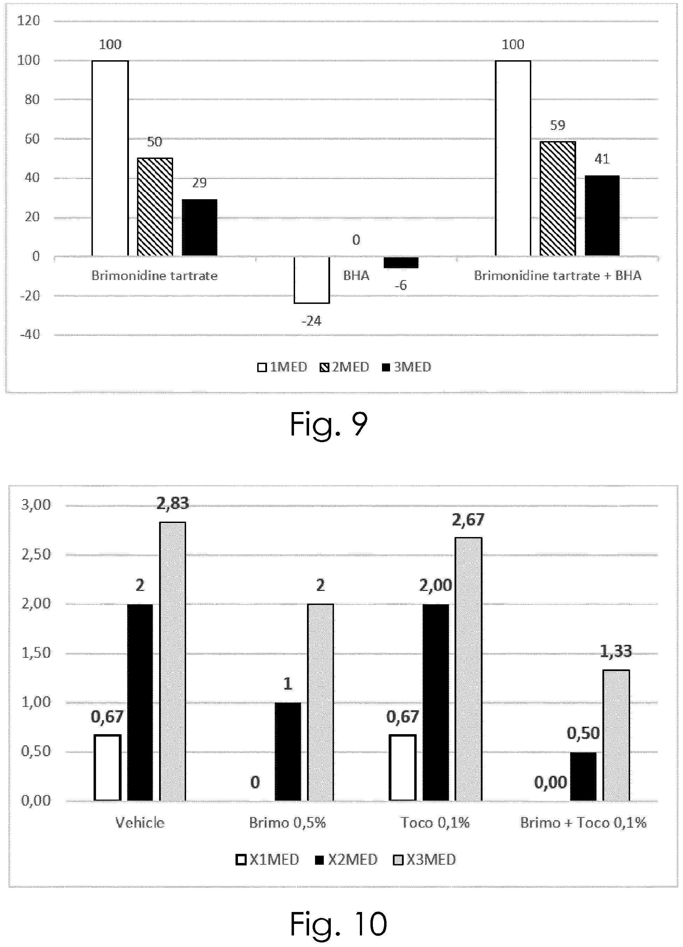

Les pourcentages de réduction de l'érythème ainsi obtenus avec la composition active (comprenant la brimonidine tartrate +/- BHA) ou avec le BHA pris seul par rapport au véhicule sont illustrés par la Figure 9.

D'après les résultats ainsi obtenus, l'antioxydant (BHA) n'a pas montré d'effet sur la réduction de l'érythème alors que la brimonidine tartrate a un effet significatif sur la réduction de l'érythème, l'effet de la brimonidine tartrate étant potentialisé de manière synergique par l'antioxydant (BHA) (effet significativement supérieur à celui de la brimonidine tartrate seule).

### Exemple 6: Mise en évidence d'un effet synergique entre la brimonidine tartrate et le tocophérol sur la réduction de l'érythème sur un modèle d'érythème induit par les UV

Sur la base du modèle et du protocole décrits dans l'exemple 4, les scores moyens obtenus avec les compositions détaillées dans le Tableau 6 (et le Tableau 4), à savoir respectivement une composition active (comprenant la brimonidine tartrate +/tocophérol, 19-0155.0132/F2 et 19-0155.0134/F1) ou sans actif (ne comprenant que le véhicule +/- tocophérol, 19-0155.0132P/F1 et 19-0155.0134P/F1) sont résumés dans la Figure 10.

Tel qu'illustré par la Figure 10, en comparant les scores moyens d'érythème obtenus pour les quatre compositions testées, un effet (réduction de l'érythème) a pu être obtenu avec la composition comprenant de la brimonidine tartrate et un effet synergique peut même être observé avec la composition comprenant à la fois de la brimonidine tartrate et du tocophérol par rapport aux compositions comprenant la brimonidine tartrate ou le tocophérol pris seul.

En effet, après une irradiation 3 x MED, l'érythème a été diminué de 29% avec la composition comprenant la brimonidine tartrate sans antioxydant, et légèrement diminué par la composition comprenant le tocophérol seul (sans brimonidine tartrate) (- 6%), alors que la combinaison de brimonidine tartrate et de tocophérol a conduit de manière surprenante à une diminution de 53% de l'érythème.

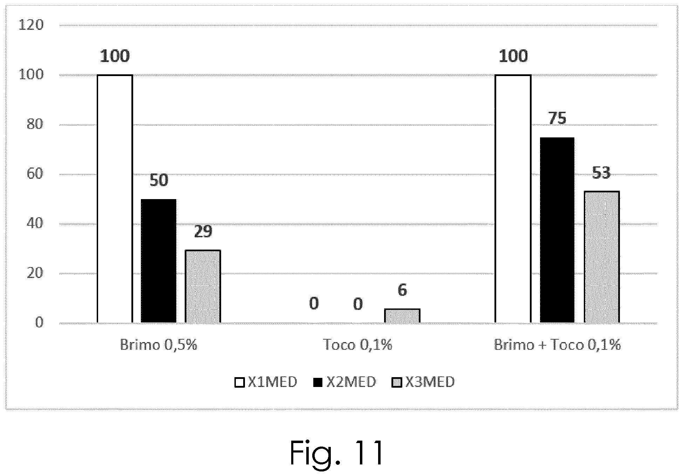

Les pourcentages de réduction de l'érythème ainsi obtenus avec la composition active (comprenant la brimonidine tartrate +/- tocophérol) ou avec le tocophérol pris seul par rapport au véhicule sont illustrés par la Figure 11.

D'après les résultats ainsi obtenus, l'antioxydant (tocophérol) n'a pas montré d'effet sur la réduction de l'érythème alors que la brimonidine tartrate a un effet significatif sur la réduction de l'érythème, l'effet de la brimonidine tartrate étant potentialisé de manière synergique par l'antioxydant (tocophérol) (effet significativement supérieur à celui de la brimonidine tartrate seule).

## Revendications

1. Composition adaptée à une administration par voie topique comprenant de la brimonidine ou ses sels, pour son utilisation dans la prévention et/ou le traitement des dommages cutanés résultant d'un rayonnement choisi parmi les ultraviolets (UV) incluant les UVA et UVB, les rayons dans le domaine du visible, le rayonnement infrarouge (IR), les rayonnements ionisants tels que les rayons X et les rayonnements alpha, beta, ou encore gamma ou les rayonnements composés de protons, **caractérisée en ce qu'**au moins une dose efficace de ladite composition est administrée à un patient subissant ledit rayonnement entre plus d'1 heure et 24 heures avant une séance de rayonnement par application au niveau d'une zone recevant ledit rayonnement.

2. Composition pour son utilisation selon la revendication 1, **caractérisée en ce que** le rayonnement est un traitement par radiothérapie ou une exposition à un rayonnement UV.

3. Composition pour son utilisation selon la revendication 1 ou 2, **caractérisée en ce qu'**elle est administrée deux fois avant la première séance de rayonnement.

4. Composition pour son utilisation selon l'une des revendications précédentes, **caractérisée en ce qu'**elle est administrée avant chaque séance de rayonnement, préférentiellement pendant toute la durée du traitement par radiothérapie.

5. Composition pour son utilisation selon l'une des revendications précédentes, **caractérisée en ce qu'**elle comprend la brimonidine ou ses sels, à une concentration comprise entre 0,15% et 1,50% en poids du poids total de la composition, préférentiellement comprise entre 0,75% et 1,50%, plus préférentiellement entre 1,00% et 1,50%, encore plus préférentiellement de 1,00% ou 1,50% p/p.

6. Composition pour son utilisation selon la revendication 5, **caractérisée en ce que** le sel de brimonidine est la brimonidine tartrate.

7. Composition pour son utilisation selon l'une des revendications précédentes, **caractérisée en ce que** la composition est sous forme d'hydrogel, émulsion huile dans eau ou émulsion eau dans huile, préférentiellement hydrogel et émulsion huile dans eau.

8. Composition pour son utilisation selon la revendication 7, **caractérisée en ce qu'**elle comprend une phase solvantée comprenant :
- du polyéthylène glycol en combinaison avec du propylène glycol et/ou du diméthylsulfoxyde ;
- un copolymère Polyvinylpyrrolidone/Vinyl Acetate comme agent filmogène hydrophile ;
- de la glycérine ;
et **en ce qu'**elle se présente sous forme d'hydrogel.

9. Composition pour son utilisation selon la revendication 8, **caractérisée en ce qu'**elle comprend du polyéthylène glycol à une concentration de 10% en poids du poids total de la composition en combinaison avec du propylène glycol à une concentration de 20% en poids du poids total de la composition.

10. Composition pour son utilisation selon la revendication 8 ou 9, qu'elle comprend un agent gélifiant choisi parmi la gomme xanthane et de l'hydroxyethylcellulose (HEC), pris seuls ou en combinaison.

11. Composition pour son utilisation selon l'une des revendications 8 à 10, **caractérisée en ce qu'**elle comprend de l'alcool oléique et de la vitamine E, pris seuls ou en combinaison.

12. Composition pour son utilisation selon l'une des revendications 8 à 11, **caractérisée en ce qu'**elle ne comprend pas de dioxyde de titane.

13. Composition pour son utilisation selon l'une des revendications précédentes, dans la prévention et/ou le traitement de la radiodermite ou d'un érythème actinique.
